# FASCICULE DE BREVET EUROPEEN

(11) **EP 0 585 151 B1**
(45) Date de publication et mention de la délivrance du brevet: **05.01.2000**
(21) Numéro de dépôt: 93401874.8
(22) Date de dépôt: 20.07.1993
(51) Int. Cl.: A61K 38/00, A61K 9/16

(54) **Procédé de préparation de microsphères pour la libération prolongée de l'hormone LHRH et ses analogues, microsphères et formulations obtenues**
Verfahren zum Herstellen von Microkugeln für die verzögerte Verabreichung des LHRH-Hormons und dessen Analoga, Microkugeln und so erhaltene Zusammensetzungen
Process to prepare micropheres for the sustained release of LHRH-hormone, microspheres and so-obtained compositions

(30) Priorité: 27.07.1992 FR 9209241
(43) Date de publication de la demande: 02.03.1994
(73) Titulaire: MERIAL, 69002 Lyon (FR)
(72) Inventeur: Billot, Geneviève Bernadette, F-69006 Lyon (FR); Teichner, Marc Maurice, F-69110 Sainte Foy les Lyon (FR)
(74) Mandataire: Bernasconi, Jean

(56) Documents cités:
- EP-A- 0 102 265
- EP-A- 0 330 180
- WO-A-89/05632
- WO-A-91/12882
- WO-A-91/13595

## Description

L'invention concerne la préparation de microsphères injectables pour la libération prolonqée de l'hormone LHRH ou de ses analogues. La préparation de ces microsphères fait appel à des polymères ou copolymères biodégradables et biocompatibles dans lesquels le polypeptide est dispersé. L'invention concerne aussi des microsphères et des formulations à libération prolongée susceptibles d'être obtenues par ce procédé.

On entend par microsphères des particules dans lesquelles le polypeptide est dispersé dans la matrice polymère. Ces particules sont injectables chez l'animal ou l'homme après mise en suspension dans un liquide adapté.

Les polymères biodégradables et biocompatibles utilisés sont généralement des poly (D.L lactide-glycolide) produits par polymérisation par ouverture de cycle, des poly (acides lactique-glycolique) produits par polycondensation de l'acide lactique et de l'acide glycolique, des polycaprolactones et copolymères, polyacétals, polyorthoesters, polyhydroxybutyrates et copolymères.

De nombreuses méthodes de microencapsulation de polypeptides hydrosolubles dans des polymères biodégradables ont été décrites. Ces méthodes se classent essentiellement en trois groupes :
- coacervation ou technique d'émulsion-séparation de phase,
- encapsulation par atomisation ou spray-drying, et
- évaporation de solvant en phase organique ou aqueuse.

La technique de coacervation ou émulsion-séparation de phase (brevets US-A-4.675.189, US-A-4.835.139, demande de brevet européen EP-A-O.302.582) a lieu en phase organique. Le peptide est dispersé en solution aqueuse ou sous forme pulvérulente dans une solution organique du polymère. Un inducteur de phase, généralement une huile silicone, est ajouté à la phase organique pour induire la coacervation du polymère sous forme de gouttelettes de coacervat enrobant le polypeptide. Ces gouttelettes fusionnent entre elles pour donner des microsphères embryonnaires. Ces microsphères sont ensuite transvasées dans un non-solvant du polymère pour induire leur durcissement. Les polypeptides employés étant généralement insolubles dans les solvants organiques et les huiles utilisés, la fraction encapsulée dans le polymère est élevée.

Cette méthode présente toutefois l'inconvénient de consommer des quantités importantes de solvants (dichlorométhane, heptane ou trichloro-trifluoro-éthane) et d'huile. Cela entraîne des coûts élevés de production et la nécessité de se prémunir contre les effets toxiques des solvants et les risques d'inflammation dans le cas de l'heptane. L'étape de coacervation est en outre délicate à maîtriser : l'obtention des microsphères individualisées est fonction des quantités de polymère, de solvant et d'inducteur de phase dans le mélange. De faibles excès d'inducteur de phase entraînent l'agrégation partielle ou totale des microsphères, rendant inutilisable le produit obtenu. D'autre part, la cinétique de libération du polypeptide encapsulé dans un milieu physiologique est caractérisée par une forte quantité libérée pendant les premières heures. Cette propriété peut entraîner in vivo des effets secondaires gênants lorsque le produit est utilisé à des fins curatives, ou peut correspondre à une perte de principe actif.

La technique d'encapsulation par atomisation décrite par exemple dans la demande de brevet EP-A-O.315.875 consiste à préparer une émulsion du peptide en solution aqueuse dans un mélange de polymères en solution organique, puis à pulvériser cette émulsion dans un flux d'air chaud. On obtient des microsphères suite à l'évaporation des solvants au cours de la pulvérisation.

La technique de base d'évaporation de solvant consiste à disperser une solution de polymère contenant un principe actif dans un deuxième solvant non miscible à celui du polymère, puis à évaporer le solvant du polymère. Une technique particulièrement utilisée avec les polymères cités plus haut est la technique d'évaporation de solvant en phase aqueuse : une solution du polymère contenant le principe actif est dispersée dans une solution aqueuse sous agitation. Le solvant du polymère est progressivement éliminé par diffusion dans la phase aqueuse, puis évaporation à la surface du mélange. On peut alors filtrer et récupérer les microsphères ainsi solidifiées. Cette technique est particulièrement utilisée lorsque le principe actif que l'on désire encapsuler est insoluble dans la phase aqueuse. Cette technique est par exemple utilisée avec succès pour l'encapsulation et la libération prolongée de stéroïdes (T. Tice, L.R. Beck, in Dr. Mishell Jr. Editor, Long Acting Steroid Contraception, Raven Press, New York, 1983, 175-199).

Le brevet US-A-4.389.330 décrit par exemple un procédé de préparation de microcapsules dans lequel on dissout ou disperse la substance à encapsuler dans un solvant peu miscible à l'eau, on y dissout le matériau formateur de paroi, on disperse la phase organique obtenue dans un milieu de traitement à phase continue qui peut être de l'eau ou un liquide organique tel que xylène, toluène, etc., puis on évapore une partie du solvant avant de récupérer les microcapsules et d'extraire le reste du solvant qu'elles contiennent. Ce procédé n'est mis en oeuvre dans cette demande que pour des substances insolubles dans l'eau, progestérone et norgestimate, dissoutes dans du chlorure de méthylène comme solvant, puis dispersées dans une phase aqueuse à 5 % de PVA (alcool polyvinylique). Aucune différence n'est faite entre les types très différents de milieux de traitement à phase continue et entre les différents solvants, par exemple THF et chlorure de méthylène, qui peuvent pourtant avoir des comportements très différents.

A l'opposé, la technique d'évaporation de solvant par dispersion d'une phase organique dans une phase aqueuse procure de faibles rendements d'encapsulation lorsque le principe actif est hydrosoluble. Dans ce cas, le principe actif dissous ou dispersé dans la solution organique peut rapidement diffuser et se dissoudre dans la phase aqueuse (Bodmeier R., Mc Ginity J.W., Pharm. Res., 1987, 4, 465). Ces microsphères ainsi produites ne contiennent qu'une faible proportion de principe actif initial : la majeure partie du principe actif est perdue par dissolution dans la phase aqueuse.

Les inconvénients de la technique classique d'évaporation de solvant ont conduit le spécialiste à rechercher et mettre au point, pour l'encapsulation de substances hydrosolubles, des techniques toujours plus complexes et coûteuses (coacervation : EP-A-O.302.582, US-A-4.675.189, US-A-4.835.139 atomisation ; double émulsion : EP-A-0.145.240, EP-A-0.442.671) ou à adapter l'évaporation de solvant à des cas particuliers de produits hydrosolubles.

Dans le cas où la solubilité du principe actif dépend du pH, un ajustement du pH de la phase aqueuse à une valeur correspondant à une faible solubilité du principe actif peut permettre de limiter la partition du principe actif dans la phase aqueuse. Mais, cet ajustement peut être problématique lorsqu'il faut ajuster le pH de la phase aqueuse à des valeurs extrêmes. Dans ces conditions, le principe actif et le polymère peuvent être instables.

Une autre méthode utilisée en vue de limiter la partition du principe actif dans la phase aqueuse consiste à préalablement saturer la phase aqueuse avec le principe actif en vue de supprimer ou inverser le phénomène (R. Bodmeier J.W., Mc Ginity, J. Microencapsulation, 1987, Vol. 4, n° 4, 289-297). Cette dernière solution reste toutefois inutilisable pour l'encapsulation de peptides ou polypeptides hydrosolubles de coût élevé, car les quantités nécessaires de principe actif à utiliser pour saturer la phase aqueuse sont trop importantes.

Une variante de la technique d'encapsulation par évaporation de solvant a été développée pour l'encapsulation de peptides hydrosolubles analogues de la LHRH dans des poly (acides lactique-glycolique) produits par polycondensation (demande de brevet européen EP-A-0.145.240, Chem. Pharm. Bull. 36 (3), 1095-1103, 1988), pour l'encapsulation de TRH (Int. J. Pharm., 1991, 69, 69-75), l'encapsulation de somatostatine dans des poly-(D.L. lactide-glycolide) branchés sur du D.glucose (Proceed. Intern. Symp. Control. Rel. Bioact. Mater. 18, 1991, 597-598), l'encapsulation d'albumine bovine et de peroxydase de Raifort (Pharm. Research, vol. 8, n° 6, 713-720, 1991). Cette variante consiste à former une première émulsion de type eau/huile dans laquelle le principe actif en solution dans l'eau est émulsionné dans le polymère en solution dans un solvant organique, généralement le dichlorométhane. La phase aqueuse de cette première émulsion peut également contenir un additif hydrosoluble ayant pour effet d'augmenter sa viscosité. L'augmentation de viscosité peut être due soit à un composé macromoléculaire hydrosoluble tel que la gélatine (EP-A-0.145.240), soit à l'interaction ionique existant entre le peptide et le polymère (Int. J. Pharm., 1991, 69, 69-75).

Cette première émulsion est alors dispersée dans une phase aqueuse contenant un stabilisateur de la dispersion, pour donner une émulsion du type eau/huile/eau. Le solvant de la phase organique est évaporé sous vide, pour induire le durcissement des microsphères. Les microsphères sont ensuite récoltées par centrifugation ou filtration.

La fraction de peptide ou polypeptide effectivement encapsulée par cette méthode est généralement élevée.

La cinétique de libération du principe actif dépend largement des conditions d'encapsulation, essentiellement la quantité d'eau, de principe actif et d'additif dans la phase aqueuse de la première solution, par rapport à la quantité de polymère et de solvant dans la phase organique. La réalisation de cette première émulsion est délicate dans le cas de l'analogue LHRH (EP-A-0.145.240) puisqu'elle implique l'émulsification de la phase aqueuse dans la phase organique à une température élevée, d'où la nécessité d'opérer sous une pression supérieure à la pression atmosphérique, pour éviter l'ébullition et l'évaporation de ce solvant. D'autre part, la température élevée (69 à 70°C) employée au cours de la formation de la première émulsion ne peut être utilisée pour des principes actifs thermiquement instables.

Une autre variante de la technique d'évaporation de solvant consiste à réaliser la dispersion d'une phase organique dans une autre phase organique non miscible. Cette variante permet théoriquement de limiter la partition d'un principe actif hydrosoluble de la phase dispersée vers la phase continue. Cette technique de dispersion-évaporation de solvant huile/huile consiste à disperser sous agitation une solution organique du polymère contenant le principe actif dans une huile minérale ou un second solvant organique non miscible au solvant du polymère. Le polymère est par exemple dissous dans l'acétone ou l'acétonitrile, puis la solution obtenue est dispersée dans l'huile de paraffine. D'autres couples de solvants ont été décrits comme par exemple le couple hexafluoroacétone/tétrachlorure de carbone. Toutefois, l'utilisation de solvants toxiques ou de quantités importantes d'huile de paraffine limitent la portée de ce procédé.

Par ailleurs pour la préparation de microsphères comprenant, dispersée dans une matrice de polymère, une substance hydrosoluble telle qu'une enzyme, le brevet US-A-3.691.090 prévoit lui de disperser la substance dans un solvant organique miscible ou peu miscible à l'eau et dans lequel le polymère est dissous, puis de mettre en suspension cette phase organique dans une solution aqueuse comprenant un sel inorganique empêchant la solubilisation du solvant pour permettre la séparation de phase.

Dans le brevet US-A-3.737.337, le procédé prévoit de disperser une substance, soluble ou non dans l'eau, dans une solution d'un solvant organique soluble à raison d'au plus 15 % en poids dans l'eau à 20°C, puis de disperser la phase organique obtenue dans une phase aqueuse saturée en solvant organique ou en sels de façon à empêcher dans un premier temps la solubilisation du solvant, puis d'induire progressivement la partition du solvant de la phase organique vers la phase aqueuse par addition progressive d'eau.

Enfin, la demande de brevet internationale WO 91/12882 résume bien l'état des connaissances en matière d'encapsulation de peptides hydrosolubles : la technique par évaporation de solvant est reconnue traditionnellement comme inadaptée pour ceux-ci ; la méthode par coacervation est la méthode reconnue comme étant la mieux adaptée.

Cette demande internationale propose toutefois d'adapter l'évaporation de solvant ou émulsion/évaporation en choisissant une nouvelle voie qui consiste à solubiliser le peptide hydrosoluble dans un tiers solvant éventuellement additionné d'eau et utilisé conjointement au solvant habituel tel que le dichlorométhane. Le tiers solvant est miscible à l'eau. Lorsque la phase organique est dispersée dans la phase aqueuse, le dichlorométhane est évaporé tandis que le tiers solvant passe dans la phase aqueuse. Si le rendement d'encapsulation peut dépasser les 90 % dans le cas de certaines substances peptidiques telles que la calcitonine de saumon, le TPA (Tissue Plasminogen Activator) et l'insuline, les rendements observés pour l'encapsulation de l'hormone LHRH sont de l'ordre de 75 %. Ces résultats traduisent une partition non négligeable de l'hormone LHRH avec le tiers solvant.

Un autre inconvénient important de ce procédé réside dans l'utilisation de volumes encore importants de solvants difficiles à extraire, ce qui se traduit par un taux résiduel de solvant pouvant tout de même atteindre 1,5 % du poids de la microsphère. En outre, notamment lorsque le dichlorométhane est utilisé, un troisième solvant, tel que l'éthanol, doit être ajouté à la phase aqueuse, en quantité pouvant atteindre 20 % en volume.

La présente invention a pour objectif de fournir un procédé simple pour encapsuler l'hormone LHRH et ses analogues avec des rendements satisfaisants, notamment supérieurs à 80 % et plus particulièrement supérieurs à 90 %, et nécessitant de faibles volumes de solvants pour l'obtention de microsphères à faible taux de résidus.

Un autre objectif de l'invention est de proposer un procédé permettant d'obtenir des microsphères pour une libération prolongée de LHRH ou de ses analogues sur une durée pouvant aller de quelques jours à environ 1 an lorsqu'elles sont injectées à un sujet ou placées in vitro dans un tampon physiologique.

L'invention a donc pour objet un procédé de préparation de microsphères pour la libération prolongée de l'hormone LHRH et de ses analogues, l'hormone étant dispersée dans une matrice polymérique ou copolymérique insoluble dans l'eau, procédé dans lequel on disperse l'hormone et l'on dissout la matière destinée à former la matrice dans un solvant organique, on met en suspension la phase organique ainsi obtenue dans une phase aqueuse continue, on évapore le solvant organique et l'on récupère les microsphères formées, caractérisé en ce qu'on disperse l'hormone à l'état pulvérulent dans un couple de solvants organiques dont l'un, dit solvant de dispersion, permet plus spécifiquement d'obtenir une suspension homogène de l'hormone à l'état pulvérulent par simple agitation et dont l'autre, dit deuxième solvant, est légèrement miscible à l'eau pour permettre plus spécifiquement la microdispersion de la phase organique dans la phase aqueuse. Il est remarquable que le solvant légèrement miscible à l'eau diminue la partition de l'hormone, ce qui semble dû notamment à un début de durcissement superficiel de la matrice suite à la fuite de ce solvant.

Par hormone LHRH (Luteinizing Hormone Releasing Hormone, encore appelée GnRH pour hormone gonadolibérine), on entend l'hormone naturelle ou de synthèse, d'origine humaine ou animale. Par analogues de la LHRH, on entend notamment les fragments, les agonistes et les antagonistes de LHRH et leurs sels. Dans les revendications et les parties correspondantes de la description, lorsqu'on parle de l'hormone (ou hormone LHRH) de manière générale, il faut bien entendu entendre l'hormone elle-même telle que définie ci-dessus, mais aussi ses analogues.

De façon très avantageuse, ce procédé utilise de faibles quantités de solvants par rapport aux procédés classiques d'encapsulation de peptides hydrosolubles. Il permet en outre d'obtenir une très bonne dispersion de l'hormone et de ses analogues sans nécessiter les techniques d'agitation vigoureuses habituellement utilisées. En outre, il évite l'utilisation en quantité de substances additionnelles susceptibles de se retrouver en plus ou moins grande quantité dans les microsphères, telles que gélatine, huile de silicone, sels organiques ou inorganiques, ainsi que l'utilisation de solvants organiques à caractère toxique, tels que l'heptane, indésirables à la fois comme constituants involontaires des microsphères et comme résidus du procédé.

La viscosité de la phase organique est aussi un paramètre important et l'on préfère qu'elle soit comprise entre 0,01 et 10 Pa.s, de préférence entre 0,01 et 1 Pa.s. et notamment supérieure à 0,04 Pa.s environ.

Selon un premier mode de réalisation du procédé, on dissout la matière destinée à former la matrice dans le solvant de dispersion, où l'on disperse ensuite l'hormone sous agitation, on évapore en tout ou partie, de préférence en totalité, le solvant de dispersion, on reprend le restant par le deuxième solvant et l'on procède à la mise en suspension de la phase organique dans la phase aqueuse.

Selon un deuxième mode de réalisation du procédé, on disperse dans un premier temps l'hormone dans le solvant de dispersion tandis que l'on dissout la matière destinée à former la matrice dans le deuxième solvant, puis on mélange les deux phases obtenues pour obtenir la phase organique que l'on met en suspension dans la phase aqueuse.

Le solvant de dispersion est choisi de préférence parmi les solvants tétrahydrofuranne (THF), acétone, dichlorométhane, chloroforme, toluène, méthyléthylcétone, pyridine, alcool benzylique, acétonitrile, acétate d'éthyle, dioxane, mélanges de ceux-ci, ou encore les solvants chlorofluorocarbonés, tandis que le deuxième solvant est avantageusement du dichlorométhane ou encore du chloroforme.

Dans des cas avantageux, un solvant unique peut assurer la mise en oeuvre adéquate du procédé selon l'invention. Il est plus simple alors de disperser l'hormone dans le solvant dans lequel le polymère a été préalablement dissout. Ainsi, le deuxième mode de réalisation peut mettre en oeuvre un solvant unique tel que le dichlorométhane. Le dichlorométhane disponible dans le commerce est généralement stabilisé par l'éthanol (par exemple à 0,3 % d'éthanol pour le dichlorométhane pur pour synthèse de SDS). L'invention utilise indifféremment ce solvant à l'état stabilisé ou non.

De préférence, la matière destinée à former la matrice fait appel à des poly(lactide-glycolide), polylactides, acides polylactiques, poly(acides lactique-glycolique), polycaprolactones, polyvalerolactones, polyhydroxybutyrates, poly(hydroxybutyrate-valérate) ainsi qu'aux mélanges de ces polymères. Les polymères, en particulier les polymères préparés à partir de l'acide lactique et de l'acide glycolique, ou à partir des dimères cycliques lactide et glycolide, génèrent au cours de leur dégradation des produits non toxiques métabolisés par l'organisme. La vitesse de dégradation de ces polymères est un facteur permettant de contrôler la cinétique de libération du principe actif, qu'il est notamment possible de faire varier de quelques jours à un an environ.

Le solvant de dispersion est de préférence évaporé sous vide, avantageusement en totalité. Après reprise par le deuxième solvant, la phase organique peut être injectée à vitesse constante dans la phase aqueuse maintenue sous agitation. Cette phase aqueuse comporte de préférence un stabilisant de dispersion tel que alcool polyvinylique (PVA) (notamment moins de 5 %, en particulier entre 0,5 et 2 %), gélatine, ou un tensio-actif, tel que "Tween 80". Le solvant organique contenu dans les microsphères en suspension dans la phase aqueuse est de préférence évaporé progressivement en faisant circuler de l'air comprimé (bullage d'air) dans la phase aqueuse maintenue sous agitation. Quelques gouttes d'un agent antimousse, tel que notamment une émulsion silicone, peuvent avantageusement être ajoutées dans la phase aqueuse pour éviter la formation de mousse due au bullage d'air comprimé.

Après évaporation du solvant, les microsphères obtenues peuvent être récupérées par filtration, lavées à l'eau déminéralisée, puis éventuellement lavées à l'aide d'un non-solvant tel que trichloro-trifluoro-éthane, heptane, éther de pétrole. On obtient ainsi une poudre s'écoulant très librement, notamment de granulométrie inférieure à 250 µm.

Le procédé selon l'invention permet d'incorporer l'hormone LHRH ou ses analogues avec un rendement élevé en mettant en oeuvre de faibles quantités de solvants organiques.

Le procédé selon l'invention permet de conserver la substance en son état pulvérulent initial jusqu'à la formation des microsphères dans lesquelles elle se trouve dispersée de façon homogène, sans agrégation, ce qui, allié à la partition initiale du deuxième solvant, permet de limiter de façon considérable les pertes de substance qui autrement se produiraient par solubilisation dans la phase aqueuse.

Le rendement d'encapsulation peut encore être amélioré en ajustant la température de la phase aqueuse entre 0 et 30° C environ et en particulier entre 10 et 25°C.

Le procédé selon l'invention est le premier procédé par évaporation de solvant permettant d'obtenir un bon rendement d'encapsulation de l'hormone LHRH et de ses analogues. Les microsphères ainsi obtenues sont remarquables notamment par le fait que l'hormone est dispersée de façon très homogène et dans son état pulvérulent initial, le procédé permettant d'éviter également l'agrégation des particules. En outre, comme ceci a été précisé ci-dessus, les microsphères obtenues ont un faible taux de substances résiduelles indésirables. Enfin, le procédé permet d'obtenir une large gamme de microsphères incorporant l'hormone LHRH ou ses analogues tant du point de vue de la durée de libération qui peut aller de quelques jours à environ 1 an, que du point de vue de leurs dimensions, celles-ci pouvant dépasser notamment les 50 à 60 microns, dimensions non atteintes par les techniques utilisées classiquement pour encapsuler des peptides hydrosolubles sous forme solide telles que la coacervation.

L'invention a donc aussi pour objet les microsphères susceptibles d'être obtenues par le procédé selon l'invention. Ces microsphères peuvent avoir des dimensions allant de 1 à 250 microns, notamment supérieures à 50 à 60 microns.

La présente invention a encore pour objet des microsphères susceptibles d'être obtenues par le procédé selon l'invention et remarquables par le fait que la matrice qui les constitue comprend au moins deux types de polymère ou copolymère, de préférence deux ou trois, qui peuvent notamment différer par leur nature ou mieux encore être de même nature mais différer par une ou plusieurs caractéristiques telles que le ratio en motifs monomères constitutifs ou leurs masses moléculaires. Cela permet de jouer sur les vitesses de libération et d'obtenir une libération continue de longue durée pouvant dépasser notamment les 6 mois. D'autres avantages importants y sont liés, tels que surtout la polydispersité du mélange final, ainsi qu'une libération continue de principe actif présentant une phase d'induction préalable fortement réduite, notamment inférieure à 5 à 10 %, voire nulle.

De préférence, la matrice comprend un mélange de deux polymères poly (D. L lactide-glycolide) de ratios différents allant de 40-60 à 100-0. On peut citer à titre d'exemple une matrice comprenant du poly (D. L lactide-glycolide) 75-25 et poly (D. L. lactide-glycolide) 50-50 et par exemple environ 360 mg et 40 mg respectivement pour une période de libération continue s'étalant de J20 à J180 environ. Les quantités respectives de chacun des types de polymères ou copolymères est bien entendu un facteur sur lequel il est aussi possible de jouer.

L'invention a encore pour objet des formulations comprenant au moins deux types de microsphères selon l'invention qui diffèrent par la composition des matrices, pour obtenir une libération continue de longue durée, notamment de l'ordre de 8 mois ou plus, et/ou à temps de latence faible ou nul. Les matrices peuvent notamment différer par leur nature ou être de même nature mais différer par leurs ratios en motifs monomères constitutifs et/ou par leurs masses moléculaires. Il est remarquable que l'on puisse égaler ou dépasser 8 mois de libération de LHRH in vitro comme in vivo à l'aide de seulement deux types de microsphères. On peut notamment obtenir, à partir de deux types de microsphères, une libération prolongée égalant ou dépassant les 8 mois à partir du jour d'administration avec un profil de libération sensiblement continu et proche de l'ordre zéro.

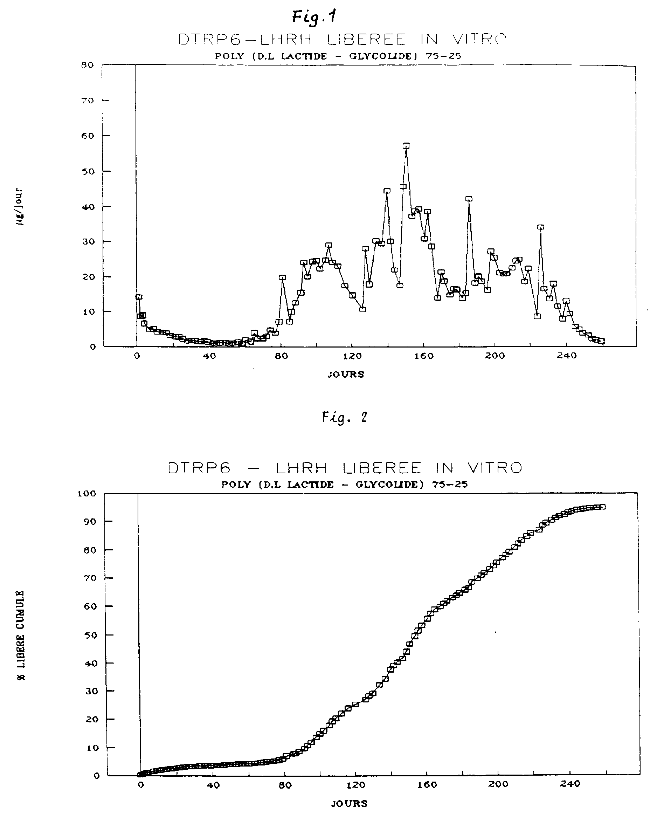

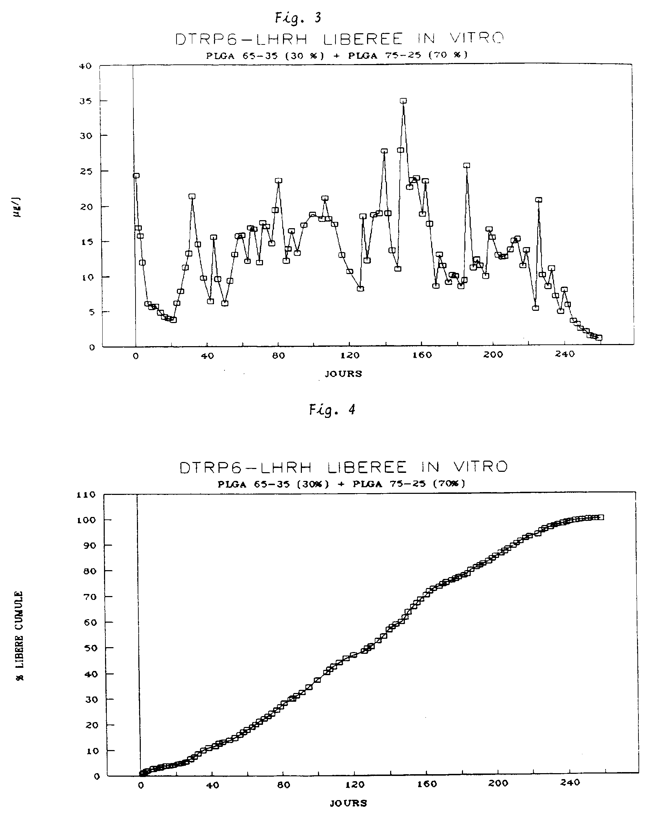

L'invention va être maintenant décrite plus en détail à l'aide des modes de réalisation du procédé selon l'invention pour la préparation de microsphères incorporant un peptide [D.Trp⁶]-LHRH et illustrant la cinétique de libération de ce peptide à l'aide du dessin annexé où l'on voit :
- aux figures 1 et 2, des graphes montrant la cinétique de libération in vitro de [D.Trp⁶]-LHRH en microsphères de poly (D.L lactide-glycolide) (= PLGA) 75-25, en µg/jour et pourcentages cumulés respectivement ;
- aux figures 3 et 4, des graphes correspondant à ceux des figures 1 et 2 pour un mélange comprenant 30 % de microsphères à PLGA 65-35 et 70% de microsphères à PLGA 75-25 ;

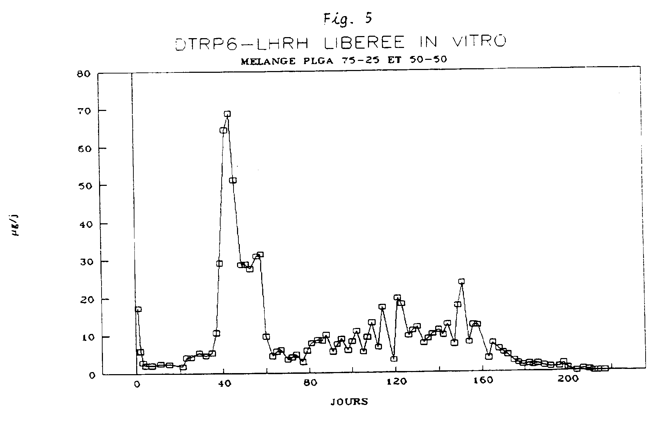

- à la figure 5, un graphe montrant la cinétique de libération en µg/jour pour des microsphères dont la matrice est un mélange de PLGA 75-25 et de PLGA 50-50.

### EXEMPLE 1

### METHODE DE PREPARATION, RENDEMENT D'ENCAPSULATION

On appelle rendement d'encapsulation le rapport de la quantité de principe actif réellement encapsulé sur la quantité totale de principe actif utilisée au début du procédé.

### 1. Préparation des microsphères

La phase organique, contenant le polymère et le principe actif, est préalablement préparée comme suit : On dissout 400 mg de poly (D.L lactide-glycolide) 75-25 de viscosité inhérente 0,59 dl/g dans 3,5 g de tétrahydrofuranne (THF). On ajoute progressivement à cette solution organique 39,6 mg d'un lyophilisat d'hormone [D.Trp⁶]-LHRH (trifluoroacétate) sous agitation. Le solvant est totalement évaporé sous vide, puis la masse est dissoute dans 2,4 g de dichlorométhane sous agitation. Cette dispersion d'hormone [D.Trp⁶]-LHRH est injectée dans 500 ml d'eau déminéralisée contenant 1 % d'alcool polyvinylique (PVA 8/88) à 19°C, sous agitation. Dès la fin de l'injection, on ajoute 3 gouttes d'antimousse (émulsion silicone). On évapore alors, toujours sous agitation, le dichlorométhane à l'aide d'un bullage d'air comprimé dans le mélange. Après évaporation du solvant, on récolte les microsphères par filtration sous vide, puis on les lave avec de l'eau déminéralisée en vue d'éliminer le PVA résiduel et une fraction du silicone antimousse. Les microsphères recueillies sont séchées de leur eau sur un filtre, puis sont lavées avec du trichloro 1, 1, 2 trifluoro 1, 2, 2 éthane pour ôter l'antimousse résiduel. Les microsphères sont alors recueillies et stockées à + 4°C. Les microsphères ainsi produites contiennent 8,1 % de [D.Trp⁶]-LHRH (9 % théorique).

### 2. Rendement d'encapsulation

L'exemple cité ci-dessus fait référence à un sel d'hormone peptidique de 10 acides aminés, de solubilité d'environ 45 mg/ml dans la phase continue. Bien que la solubilité de ce peptide dans l'eau soit élevée, on obtient de bons rendements d'encapsulation.

Un paramètre important est la viscosité de la phase organique contenant le polymère, la dispersion de peptide et le ou les solvants. On mesure la viscosité de la solution polymère/dichlorométhane à 19°C à l'aide d'un viscosimètre de type Ostwald, puis on réalise les essais d'encapsulation comme décrits à l'exemple 1 en faisant varier le volume de dichlorométhane, donc la viscosité de la phase organique. On trouve alors une relation directe entre la viscosité et le rendement d'encapsulation :

| Viscosité (Pa.s) | Rendement d'encapsulation (%) |
|---|---|
| 0,014 | 81 |
| 0,018 | 84 |
| 0,030 | 86 |
| 0,040 | 90 |

Le rendement d'encapsulation est déterminé à partir de l'extraction de l'hormone des microsphères produites, et dosage en HPLC.

Un rendement de 90 % peut également être obtenu avec des charges élevées en peptide (15 %), la charge étant définie comme le rapport de la masse de peptide sur la quantité totale polymère + peptide.

Les microsphères produites dans les conditions de rendement optimal (viscosité > 0,04 Pa.s) ont une taille < 250 µm et sont injectables après mise en suspension dans un véhicule aqueux adéquat.

Un abaissement de température de la phase aqueuse continue permet d'améliorer encore le rendement d'encapsulation : si le mélange de viscosité 0,04 Pa.s utilisé dans l'exemple 1 est injecté dans une phase aqueuse à 13°C, le rendement passe de 90 à 94 %.

Afin de former une dispersion de peptide de faible granulométrie, d'autres solvants ou mélanges de solvants sont utilisables tels que l'hormone reste sous forme dispersée sans se solvater, par exemple le chloroforme, l'acétone, l'acétate d'éthyle, etc. Ces solvants, utilisés purs ou en mélange, ont comme caractéristiques le fait que le peptide y est dispersible sous agitation modérée pour obtenir une granulométrie de quelques microns, et le fait que le polymère utilisé y est soluble.

### EXEMPLE 2

Une simplification du procédé peut être apportée si l'on réalise directement la dispersion phase organique/phase aqueuse sans évaporation préalable : On dissout 1 g de poly (D.L lactide-glycolide)75-25 cité dans l'exemple 1 dans 5,2 g de dichlorométhane (produit commercial SDS à 0,3 % d'éthanol). On disperse 98,9 mg d'un lyophilisat de [D.Trp⁶]-LHRHdans 0,53 g de dichlorométhane (éthanol 0,3 %). On mélange alors la dispersion de peptide à la solution de polymère sous agitation, puis on injecte ce mélange dans 500 ml d'eau déminéralisée à 19°C contenant 1 % de PVA 8/88 sous agitation. La suite du procédé est identique à l'exemple 1.

Le rendement d'encapsulation est de 86 %.

De manière équivalente, on disperse directement le peptide dans la solution de polymère-dichlorométhane, sous agitation modérée : on procède ensuite comme à l'exemple 2.

De manière équivalente encore, on disperse un lyophilisat de [D.Trp⁶, desGly 10, NH₂]-LHRH éthylamide dans la solution de polymère et le dichlorométhane, sous agitation modérée. De façon identique, la partition du peptide dans la phase aqueuse reste faible. Des rendements sensiblement équivalents sont obtenus.

Certains couples de solvants de dispersion du peptide peuvent être utilisés tels quels dans la phase ultérieure de dispersion de la phase organique dans la phase aqueuse (mélange dichlorométhane/chloroforme). Ceci permet donc de se passer de la phase d'évaporation du solvant de dispersion citée à l'exemple 1 lorsque celui-ci n'est pas ou peu miscible à l'eau (chloroforme,...).

L'ordre d'incorporation du peptide ou du polymère dans le solvant de dispersion peut être interchangé tout en restant conforme à l'invention.

### EXEMPLE 3

### PREPARATION DE MICROSPHERES A PARTIR DE POLY (D.L LACTIDE-GLYCOLIDE) 65-35

On dissout 2 g de poly (D.L lactide-glycolide) 65-35 de viscosité inhérente 0,69 dl/g dans 8,9 g de THF. On ajoute à cette solution 198 mg d'un lyophilisat de [D.Trp⁶]-LHRH sous agitation. Le solvant est évaporé sous vide et sous agitation, puis le résidu sec est dissous dans 11,8 g de dichlorométhane sous agitation. La suspension obtenue est injectée dans 500 ml d'eau déminéralisée contenant 1 % d'alcool polyvinylique 8/88 à 20°C, sous agitation mécanique (700 t/min). La suite du procédé est identique à l'exemple 1. On obtient des microsphères de taille < 250 µm, formant une poudre s'écoulant librement. Le rendement d'encapsulation est de 86 %.

### EXEMPLE 4

### PREPARATION DE MICROSPHERES A PARTIR DE POLY (D.L LACTIDE-GLYCOLIDE) 75-25 : LIBERATION DE L'AGONISTE [D.Trp⁶]-LHRH DE 80 A 240 JOURS

On utilise les microsphères produites à l'exemple 1, contenant 8,1 % de [D.Trp⁶]-LHRH.

On immerge 50 mg de microsphères dans 5 ml de tampon phosphate pH 7,2, isotonique, à 37°C. On prélève le surnageant régulièrement et on le remplace par 5 ml de tampon à 37°C. Chacun des prélèvements est ensuite analysé par HPLC, pour déterminer la quantité d'hormone [D.Trp⁶]-LHRH libérée en fonction du temps.

La cinétique de libération in vitro, comparée à la cinétique in vivo, a permis de montrer leur parallélisme. Les durées et profils de libération sont notamment identiques in vitro et in vivo.

Pour des microsphères chargées à 8,1 % de [D.Trp⁶]-LHRH, préparées à partir du poly (D.L lactide-glycolide) 75-25, on obtient, pour 50 mg de microsphères, la cinétique de libération suivante (cf. fig. 1 et 2).
Fig. 1 : Après un faible pic initial de libération de l'hormone, représentant environ 2 % de la quantité totale d'hormone, la libération in vitro est faible jusqu'à environ J80 correspondant à la phase d'induction, puis la libération augmente progressivement pour se stabiliser à environ 20 µg/j jusqu'au 220e jour. La libération décroît ensuite pour s'annuler vers le 260e jour.
Fig.2 : La fraction cumulée d'hormone libérée in vitro montre que la libération est proche de l'ordre zéro de J80 à J240.

### EXEMPLE 5

### LIBERATION CONTINUE D'HORMONE [D.Trp⁶]-LHRH PENDANT 8 MOIS A PARTIR DU MELANGE DE PLUSIEURS FORMULATIONS

Si on mélange les formulations produites dans l'exemple 1 et dans l'exemple 3, on peut ainsi obtenir un système de libération continue de l'hormone[D.Trp ⁶]-LHRH pendant 8 mois.

Les essais de libération in vitro réalisés en mélangeant 30 % (sur la base de la quantité de peptide encapsulé dans chacune des formulations) de la formulation poly (D.L lactide-glycolide) 65-35 et 70 % de la formulation poly (D.L lactide-glycolide) 75-25 dans les mêmes conditions qu'à l'exemple 4, montrent qu'il y a effectivement additivité et par conséquent complémentarité des deux formulations : la formulation PLGA 65-35 libère l'hormone de 0 à 85 jours, puis la formulation PLGA 75-25 libère l'hormone de 80 à 240 jours (fig. 3). La libération d'hormone suit une cinétique proche de l'ordre 0 jusqu'à J240 (fig.4).

### EXEMPLE 6

### LIBERATION CONTINUE D'HORMONE [D.Trp⁶]-LHRH PENDANT 6 MOIS A PARTIR DU MELANGE DE 2 POLYMERES DANS UNE FORMULATION UNIQUE

En vue d'obtenir une libération continue d'hormone pendant de longues durées (6 mois ou davantage), une autre méthode consiste à mélanger deux, voire trois, polymères de caractéristiques différentes au sein d'une même formulation :
- soit deux polymères ayant le même ratio D.L lactide-glycolide, mais des masses moléculaires différentes, de telle sorte que la polydispersité du mélange final soit élevée, par exemple située entre 3,5 et 30.
- soit deux polymères ayant des ratio D.L lactide-glycolide différents, de telle sorte que la vitesse de dégradation de l'un et l'autre de ces polymères soit différente, et permette la libération continue du principe actif sans phase d'induction préalable.

On a réalisé ce type de mélange dans l'exemple suivant :

On dissout 360 mg de poly (D.L lactide-glycolide) 75-25 (viscosité inhérente 0,59 dl/g) et 40 mg de poly (D.L lactide-glycolide) 50-50 (viscosité inhérente 0,44 dl/g) dans 3,5 g de THF. On ajoute à cette solution 16,7 mg d'un lyophilisat de [D.Trp⁶]-LHRH (trifluoroacétate) sous agitation. Ce solvant est alors évaporé sous agitation, puis le résidu sec est dissous dans 2,4 g de dichlorométhane. La suspension obtenue est injectée dans 500 ml d'eau déminéralisée contenant 1 % d'alcool polyvinylique 8/88 à 20°C, sous agitation mécanique (700 t/min). La suite du procédé est identique à l'exemple 1. On obtient des microsphères de taille ≤ 250 µm, formant une poudre s'écoulant librement. Le rendement d'encapsulation est de 93 %.

### CINETIQUE DE LIBERATION IN VITRO

On immerge 50 mg des microsphères produites à l'exemple 6 dans 5 ml de tampon phosphate pH 7,2 à 37°C, puis on prélève régulièrement le surnageant de façon identique à l'exemple 4.

On observe (fig.5) un net raccourcissement de la phase d'induction, qui passe de 80 jours (cf. exemple 4) à 20 jours. Cette phase est suivie d'une période de libération continue de l'hormone de J20 à J180.

Le mélange de polymères de caractéristiques différentes permet donc de réduire la durée de la phase d'induction et permet la libération continue du principe actif pendant environ 160 jours dans l'exemple présent.

## Revendications

1. Procédé de préparation de microsphères pour la libération prolongée de l'hormone LHRH et de ses analogues, ces microsphères étant formées d'une matrice polymérique ou copolymérique insoluble dans l'eau comprenant l'hormone ou analogue, procédé dans lequel on forme une phase organique comprenant l'hormone LHRH ou analogue et la matière destinée à former la matrice, on met en suspension cette phase organique dans une phase aqueuse continue, on évapore le solvant organique et l'on récupère les microsphères formées, caractérisé en ce que l'on forme la phase organique en dispersant l'hormone LHRH ou analogue à l'état pulvérulent, et en dissolvant le polymère ou le copolymère, dans un solvant organique légèrement miscible à l'eau permettant cette dispersion de l'hormone à l'état pulvérulent.

2. Procédé selon la revendication 1, caractérisé en ce que le solvant est choisi dans le groupe consistant en dichlorométhane, chloroforme et mélange de ceux-ci.

3. Procédé selon la revendication 1 ou 2, caractérisé en ce qu'on disperse l'hormone LHRH à l'état pulvérulent dans un couple de solvants organiques comprenant, outre le solvant légèrement miscible à l'eau, un solvant dit solvant de dispersion, qui permet plus spécifiquement d'obtenir une suspension homogène de l'hormone ou analogue à l'état pulvérulent par simple agitation.

4. Procédé selon la revendication 3, caractérisé en ce que le solvant de dispersion est choisi parmi les solvants tétrahydrofuranne (THF) , dioxane, acétone, acétonitrile, acétate d'éthyle, dichlorométhane, chloroforme, toluène, pyridine, alcool benzylique, méthyléthylcétone, mélanges de ceux-ci, solvants chlorofluorocarbonés.

5. Procédé selon la revendication 3 ou 4, caractérisé en ce qu'on dissout la matière destinée à former la matrice dans le solvant de dispersion, où l'on disperse ensuite l'hormone, on évapore en tout ou partie le solvant de dispersion, on reprend le restant par le solvant légèrement miscible à l'eau et l'on procède à la mise en suspension de la phase organique dans la phase aqueuse.

6. Procédé selon la revendication 3 ou 4, caractérisé en ce qu'on disperse dans un premier temps l'hormone dans le solvant de dispersion tandis que l'on dissout la matière destinée à former la matrice dans le solvant légèrement miscible à l'eau, puis on mélange les deux phases obtenues pour obtenir la phase organique que l'on met en suspension dans la phase aqueuse.

7. Procédé selon la revendication 1 ou 2, caractérisé en ce qu'on disperse l'hormone dans du dichlorométhane, avant ou après dissolution du polymère dans ce même solvant, puis on met la phase organique ainsi obtenue en suspension dans la phase auqueuse.

8. Procédé selon l'une quelconque des revendications 1 à 7 caractérisé en ce que la matière destinée à former la matrice est choisie parmi les poly(lactide-glycolide), polylactides, acides polylactiques, poly(acides lactique-glycolique), polycaprolactones, polyvalerolactones, polyhydroxybutyrates, poly (hydroxybutyrates-valérate) ainsi que les mélanges de ces polymères.

9. Procédé selon l'une quelconque des revendications 1 à 8, caractérisé en ce que la phase organique comprenant la substance dispersée dans le deuxième solvant a une viscosité comprise entre 0,01 et 10 Pa.s environ, de préférence comprise entre 0,01 et 1 Pa.s, notamment supérieure ou égale à 0,04 Pa.s.

10. Procédé selon l'une quelconque des revendications 1 à 9, caractérisé en ce que la température de la phase aqueuse est ajustée entre 0 et 30°C, notamment entre 10 et 25°C.

11. Procédé selon l'une quelconque des revendications 1 à 10, caractérisé en ce qu'on évapore le solvant organique en faisant circuler de l'air comprimé dans la phase aqueuse maintenue sous agitation.

12. Procédé selon l'une quelconque des revendications 1 à 11, caractérisé en ce que la phase aqueuse comprend un stabilisant de dispersion.

13. Procédé selon l'une quelconque des revendications 1 à 12, caractérisé en ce qu'on ajoute à la phase aqueuse quelques gouttes d'un agent antimousse tel qu'une émulsion silicone.

14. Procédé selon l'une quelconque des revendications 1 à 13, caractérisé en ce que, après évaporation du solvant, les microsphères obtenues sont récupérées par filtration, lavées à l'eau déminéralisée, puis éventuellement lavées à l'aide d'un non-solvant tel que trichloro-trifluoro-éthane, heptane, éther de pétrole.

15. Procédé selon l'une quelconque des revendications 1 à 14, caractérisé en ce que l'hormone LHRH ou analogue de départ est sous forme de lyophilisat.

16. Microsphères pour la libération prolongée de l'hormone LHRH et de ses analogues, susceptibles d'être obtenues par la mise en oeuvre du procédé selon l'une quelconque des revendications 1 à 15.

17. Microsphères selon la revendication 16, caractérisée en ce que la matrice comprend au moins deux types de polymères ou copolymères, notamment deux ou trois.

18. Microsphères selon la revendication 17, caractérisées en ce que les polymères ou copolymères sont de même nature mais diffèrent entre eux par leurs ratios en motifs monomères constitutifs et/ou par leurs masses moléculaires.

19. Microsphères selon la revendication 18, caractérisées en ce que la matrice comprend un mélange de deux polymères poly (D. L lactide-glycolide) de ratios différents allant de 40-60 à 100-0.

20. Microsphères selon l'une quelconque des revendications 16 à 19, caractérisées en ce que leurs dimensions sont comprises entre 1 et 250 microns environ et notamment supérieures à 50 à 60 microns.

21. Formulation pour une libération de longue durée et/ou à temps, de latence faible ou nul, de l'hormone LHRH et de ses analogues, caractérisée en ce qu'elle comprend au moins deux types de microsphères selon l'une quelconques des revendications 16 à 20, différant par la composition des matrices.

22. Formulation selon la revendication 21, caractérisée en ce que les matrices diffèrent par leur nature, ou sont de même nature mais diffèrent par leurs ratios en motifs monomères constitutifs et/ou par leurs masses moléculaires.

23. Formulation selon les revendications 21 ou 22, caractérisée en ce qu'elle comprend deux types de microsphères pour une libération prolongée égalant ou dépassant les 6 mois.

## Claims

1. Process for the preparation of microspheres for the prolonged release of the hormone LHRH and its analogs, these microspheres being formed by a water-insoluble polymer or copolymer matrix containing the hormone or analog, process in which an organic phase is formed containing the hormone LHRH or analog and the substance intended to form the matrix, this organic phase is put into suspension in a continuous aqueous phase, the organic solvent is evaporated and the formed microspheres are recovered, characterised in that the organic phase is formed by dispersing the hormone LHRH or analog in the pulverulent state, and by dissolving the polymer or the copolymer, in an organic solvent slightly miscible with water permitting this dispersion of the hormone in the pulverulent state.

2. Process according to claim 1, characterised in that the solvent is selected in the group consisting of dichloromethane, chloroform and a mixture of the latter.

3. Process according to claim 1 or 2, characterised in that the hormone LHRH in the pulverulent state is dispersed in a pair of organic solvents containing, apart from the solvent slightly miscible with water, a solvent known as a dispersion solvent, which makes it possible more specifically to obtain a homogeneous suspension of the hormone or analog in the pulverulent state by simple agitation.

4. Process according to claim 3, characterised in that the dispersion solvent is chosen among the solvents tetrahydrofuran (THF), dioxane, acetone, acetonitrile, ethyl acetate, dichloromethane, chloroform, toluene, pyridine, benzyl alcohol, methyl ethyl ketone, mixtures of the latter, chlorofluorocarbon solvents.

5. Process according to claim 3 or 4, characterised in that the substance intended to form the matrix is dissolved in the dispersion solvent, where the hormone is then dispersed, the dispersion solvent is wholly or partially evaporated, the residue is dissolved in the solvent slightly miscible with water and the organic phase is then put into suspension in the aqueous phase.

6. Process according to claim 3 or 4,characterised in that the hormone is first dispersed in the dispersion solvent whilst the substance intended to form the matrix is dissolved in the solvent slightly miscible with water, then the two obtained phases are mixed so as to obtain the organic phase which is put into suspension in the aqueous phase.

7. Process according to claim 1 or 2, characterised in that the hormone is dispersed in dichloromethane, before or after dissolution of the polymer in this same solvent, then the organic phase thus obtained is put into suspension in the aqueous phase.

8. Process according to any one of claims 1 to 7, characterised in that the substance intended to form the matrix is selected among poly(lactide-glycolide), polylactides, polylactic acids, poly(lactic-glycolic acids), polycaprolactones, polyvalerolactones, polyhydroxybutyrates, poly(hydroxybutyrates-valerate) as well as the mixtures of these polymers.

9. Process according to any one of claims 1 to 8, characterised in that the organic phase containing the substance dispersed in the second solvent has a viscosity between approx. 0.01 and 10 Pa.s, preferably between 0.01 and 1 Pa.s, in particular greater than or equal to 0.04 Pa.s.

10. Process according to any one of claims 1 to 9, characterised in that the temperature of the aqueous phase is adjusted between 0 and 30°C, in particular between 10 and 25°C.

11. Process according to any one of claims 1 to 10, characterised in that the organic solvent is evaporated by causing compressed air to circulate in the aqueous phase kept under agitation.

12. Process according to any one of claims 1 to 11, characterised in that the aqueous phase contains a dispersion stabiliser.

13. Process according to any one of claims 1 to 12, characterised in that several drops of an antifoaming agent such as a silicone emulsion are added to the aqueous phase.

14. Process according to any one of claims 1 to 13, characterised in that, after evaporation of the solvent, the microspheres obtained are recovered by filtration, washed with demineralised water, then possibly washed with the aid of a non-solvent such as trichloro-trifluoro-ethane, heptane, petroleum ether.

15. Process according to any one of claims 1 to 14, characterised in that the hormone LHRH or starting analog is in the form of lyophilisate.

16. Microspheres for the prolonged release of the hormone LHRH and its analogs, capable of being obtained by working the process according to any one of claims 1 to 15.

17. Microspheres according to claim 16, characterised in that the matrix contains at least two types of polymers or copolymers, in particular two or three.

18. Microspheres according to claim 17, characterised in that the polymers or copolymers are of the same nature but differ from one another by their ratios of constituent monomer units and/or by their molecular masses.

19. Microspheres according to claim 18, characterised in that the matrix contains a mixture of two poly (D. L lactide-glycolide) polymers with different ratios ranging from 40-60 to 100-0.

20. Microspheres according to any one of claims 16 to 19, characterised in that their dimensions are contained between 1 and 250 microns approximately and in particular greater than 50 to 60 microns.

21. Formulation for a release of long duration and/or with a small or zero latent period, of the hormone LHRH and its analogs, characterised in that it contains at least two types of microspheres according to any one of claims 16 to 20, differing by the composition of the matrices.

22. Formulation according to claim 21, characterised in that the matrices differ by their nature, or are of the same nature but differ by their ratios of constituent monomer units and/or by their molecular masses.

23. Formulation according to claims 21 or 22, characterised in that it contains two types of microspheres for a prolonged release equal to or exceeding 6 months.

## Patentansprüche

1. Verfahren zur Herstellung von Mikrosphären zur andauernden Freisetzung des Hormons LHRH und seiner Analoga, wobei die Mikrosphären aus einer in Wasser unlöslichen polymeren oder copolymeren Matrix hergestellt werden, die das Hormon oder das Analogon umfasst, wobei bei dem Verfahren eine organische Phase hergestellt wird, die das Hormon LHRH oder das Analogon und die zur Bildung der Matrix bestimmte Substanz umfasst, diese organische Phase in einer kontinuierlichen wässrigen Phase suspendiert wird, das organische Lösungsmittel verdampft wird und die gebildeten Mikrosphären gewonnen werden, dadurch gekennzeichnet, dass die organische Phase gebildet wird, indem man in einem leicht mit Wasser mischbaren organischen Lösungsmittel, das die Dispersion des Hormons im pulverförmigen Zustand erlaubt, das Hormon LHRH oder das Analogon im pulverförmigen Zustand dispergiert und das Polymer oder Copolymer darin löst.

2. Verfahren nach Anspruch 1, dadurch gekennzeichnet, dass das Lösungsmittel ausgewählt ist aus der Gruppe, bestehend aus Dichlormethan, Chloroform und einem Gemisch davon.

3. Verfahren nach Anspruch 1 oder 2, dadurch gekennzeichnet, dass das Hormon LHRH in pulverförmigem Zustand in einem Paar organischer Lösungsmittel dispergiert wird, das außer dem leicht mit Wasser mischbaren Lösungsmittel ein als Dispersionsmittel bezeichnetes Lösungsmittel umfasst, das speziell das Erhalten einer homogenen Suspension des Hormons oder des Analogons im pulverförmigen Zustand durch einfaches Rühren ermöglicht.

4. Verfahren nach Anspruch 3, dadurch gekennzeichnet, dass das Dispersionsmittel ausgewählt ist aus den Lösungsmitteln Tetrahydrofuran (THF), Dioxan, Aceton, Acetonitril, Ethylacetat, Dichlormethan, Chloroform, Toluol, Pyridin, Benzylalkohol, Methylethylketon, Gemischen von diesem und Chlorfluorkohlenstoff-Lösungsmitteln.

5. Verfahren nach Anspruch 3 oder 4, dadurch gekennzeichnet, das die zur Bildung der Matrix vorgesehene Substanz im Dispersionsmittel gelöst wird, und dann darin das Hormon dispergiert wird, das gesamte Dispersionsmittel oder ein Teil davon verdampft wird, der Rückstand im leicht mit Wasser mischbaren Lösungsmittel aufgenommen wird und die organische Phase in der wässrigen Phase suspendiert wird.

6. Verfahren nach Anspruch 3 oder 4, dadurch gekennzeichnet, dass zunächst das Hormon im Dispersionsmittel dispergiert wird, während der zur Bildung der Matrix vorgesehene Stoff im leicht mit Wasser mischbaren Lösungsmittel gelöst wird, dann die beiden erhaltenen Phasen gemischt werden, um die organische Phase zu erhalten, die man in der wässrigen Phase suspendiert.

7. Verfahren nach Anspruch 1 oder 2, dadurch gekennzeichnet, dass das Hormon in Dichlormethan dispergiert wird, bevor oder nachdem das Polymer im gleichen Lösungsmittel gelöst wird, und dann die so erhaltene organische Phase in der wässrigen Phase suspendiert wird.

8. Verfahren nach einem der Ansprüche 1 bis 7, dadurch gekennzeichnet, dass die zur Bildung der Matrix vorgesehene Substanz ausgewählt ist aus Poly(lactid-glycolid), Polylactiden, Polymilchsäuren, Poly(milchsäure-glycolsäure), Polycaprolactonen, Polyvalerolacetonen, Polyhydroxybutyraten, Poly(hydroxybutyraten-valerat) sowie den Gemischen dieser Polymere.

9. Verfahren nach einem der Ansprüche 1 bis 8, dadurch gekennzeichnet, dass die organische Phase, die die im zweiten Lösungsmittel dispergierte Substanz umfasst, eine Viskosität von etwa 0,01 bis 10 Pa.s, vorzugsweise von 0,01 bis 1 Pa.s, insbesondere von mehr als oder gleich 0,04 Pa.s hat.

10. Verfahren nach einem der Ansprüche 1 bis 9, dadurch gekennzeichnet, dass die Temperatur der wässrigen Phase auf 0 bis 30°C, insbesondere auf 10 bis 25°C eingestellt ist.

11. Verfahren nach einem der Ansprüche 1 bis 10, dadurch gekennzeichnet, dass das organische Lösungsmittel verdampft wird, indem man Druckluft unter Rühren in der wässrigen Phase zirkulieren lässt.

12. Verfahren nach einem der Ansprüche 1 bis 11, dadurch gekennzeichnet, dass die wässrige Phase einen Dispersionsstabilisator umfasst.

13. Verfahren nach einem der Ansprüche 1 bis 12, dadurch gekennzeichnet, dass zur wässrigen Phase einige Tropfen eines Antischaummittels, wie einer Silikonemulsion, gegeben werden.

14. Verfahren nach einem der Ansprüche 1 bis 13, dadurch gekennzeichnet, dass nach dem Verdampfen des Lösungsmittels die erhaltenen Mikrosphären durch Filtration gewonnen, mit entmineralisiertem Wasser gewaschen und dann gegebenenfalls mit einem Nicht-Lösungsmittel, wie Trichlortrifluorethan, Heptan, Petrolether, gewaschen werden.

15. Verfahren nach einem der Ansprüche 1 bis 14, dadurch gekennzeichnet, dass das Ausgangs-Hormon LHRH oder -Analogon die Form eines Lyophilisates hat.

16. Mikrosphären zur andauernden Freisetzung des Hormons LHRH und seiner Analoga, die durch die Durchführung des Verfahrens nach einem der Ansprüche 1 bis 15 erhalten werden können.

17. Mikrosphären nach Anspruch 16, dadurch gekennzeichnet, dass die Matrix mindestens zwei, insbesondere zwei oder drei, Typen von Polymeren oder Copolymeren umfasst.

18. Mikrosphären nach Anspruch 17, dadurch gekennzeichnet, dass die Polymere oder Copolymere von gleicher Art sind, sich aber untereinander durch das Verhältnis der in ihnen enthaltenen Monomereinheiten und/oder durch ihre Molekülmassen unterscheiden.

19. Mikrosphären nach Anspruch 18, dadurch gekennzeichnet, dass die Matrix ein Gemisch der zwei Polymere Poly(D,L-lactid-glycolid) in verschiedenen Verhältnissen von 40-60 bis 100-0 umfasst.

20. Mikrosphären nach einem der Ansprüche 16 bis 19, dadurch gekennzeichnet, dass ihre Abmessungen von etwa 1 bis 250 Mikron reichen und sie insbesondere größer als 50 bis 60 Mikron sind.

21. Formulierung zur andauernden Freisetzung und/oder zur Freisetzung mit kleiner oder ohne Latenzzeit des Hormons LHRH und seiner Analoga, dadurch gekennzeichnet, dass sie mindestens zwei Arten von Mikrosphären nach einem der Ansprüche 16 bis 20 umfasst, die sich in der Zusammensetzung der Matrices unterscheiden.

22. Formulierung nach Anspruch 20, dadurch gekennzeichnet, dass sich die Matrices durch ihre Art unterscheiden oder von gleicher Art sind, sich aber durch das Verhältnis der ihnen enthaltenen Monomereinheiten und/oder durch ihre Molekülmassen unterscheiden.

23. Formulierung nach den Ansprüchen 21 oder 22, dadurch gekennzeichnet, dass sie zwei Arten von Mikrosphären zur andauernden Freisetzung für 6 Monate oder länger umfasst.
